**Europäisches Patentamt**

**European Patent Office**

**Office européen des brevets**

(11) Veröffentlichungsnummer : **0 167 783**
**B1**

(12) **EUROPÄISCHE PATENTSCHRIFT**

(45) Veröffentlichungstag der Patentschrift :
19.10.88

(51) Int. Cl.⁴ : **B 65 D 47/08**, B 65 D 1/02

(21) Anmeldenummer : 85106641.5

(22) Anmeldetag : 30.05.85

(54) **Verschliessvorrichtung für die Entnahmeöffnung eines Behälters und zugehöriger Behälter.**

(30) Priorität : 28.06.84 DE 3423851
22.12.84 DE 3447223
16.02.85 DE 3505454

(43) Veröffentlichungstag der Anmeldung :
15.01.86 Patentblatt 86/03

(45) Bekanntmachung des Hinweises auf die Patenterteilung : 19.10.88 Patentblatt 88/42

(84) Benannte Vertragsstaaten :
AT BE CH DE FR GB IT LI NL SE

(56) Entgegenhaltungen :
DE-A- 1 808 875
FR-A- 1 574 819
FR-A- 2 288 683
FR-A- 2 407 140
US-A- 3 283 964
US-A- 3 918 578

(73) Patentinhaber : ZELLER PLASTIK Koehn, Gräbner &
Co.
Auf dem Barl Postfach 1120
D-5583 Zell/Mosel (DE)

(72) Erfinder : Koehn, Jochen
Bergwinkel 4
D-5583 Zell/Mosel (DE)
Erfinder : Brach, Ulrich
Königsbergweg 10a
D-5583 Zell/Mosel (DE)
Erfinder : Sacherer, Klaus Dieter
Westring 17
D-6719 Kirchheim/Weinstrasse (DE)
Erfinder : Weiss, Erich
Zwerchgasse 49
D-6800 Mannheim 31 (DE)

(74) Vertreter : Schroeter, Helmut et al
Bocksgasse 49
D-7070 Schwäbisch Gmünd (DE)

**Beschreibung**

Die Erfindung bezieht sich auf eine Verschließvorrichtung der im Oberbegriff von Anspruch 1 genannten Art. Eine derartige Verschließvorrichtung ist aus dem Dokument US-A-3 283 964 — Anderson — bekannt. Die dort in den Figuren 2 und 4 dargestellte Verschließvorrichtung hat einen plattenartigen Teil 16, 17. Sie wird mit Hilfe einer Schraubhülse in eine passende Schraubhülse des Behälters geschraubt. Damit die Einfüllöffnung des Behälters abgedichtet wird, muß vollständig zugeschraubt werden. Je nach den unvermeidbaren Toleranzen dürfte dann ein zum Verschließen der Entnahmeöffnung dienender Zapfen (in Draufsicht auf die Entnahmeöffnung betrachtet) etwas links oder rechts von dieser stehen, aber nur in wenigen Ausnahmefällen in einer passenden Lage.

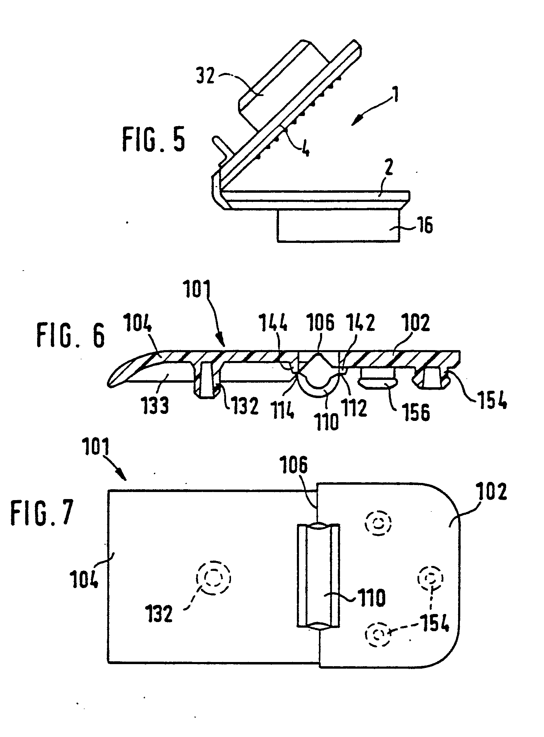

Eine Verschließvorrichtung nach den Figuren 5 und 6 des genannten Dokuments ist als Kappe ausgebildet und wird lediglich aufgesteckt. Die Verschließvorrichtung kann somit eine beliebige Stellung gegenüber dem Behälter einnehmen, und es ist nicht gesichert, daß ihr Stopfen beim Verschließen in die Entnahmeöffnung eindringt.

Durch die vorliegende Erfindung soll eine Verschließvorrichtung nach dem Oberbegriff von Anspruch 1 für eine orientierungssichere Befestigung an dem Behälter selbst oder an einer Überkappe des Behälters ausgestaltet werden, und zwar soll dies bei einer Verschließvorrichtung realisiert werden, die im Gegensatz zu dem oben genannten Dokument als Schnappscharnier ausgestaltet ist, so daß der Verschlußschenkel aus einer Stellung labilen Gleichgewichts entweder in die Schießstellung oder in die Offenstellung schnappt.

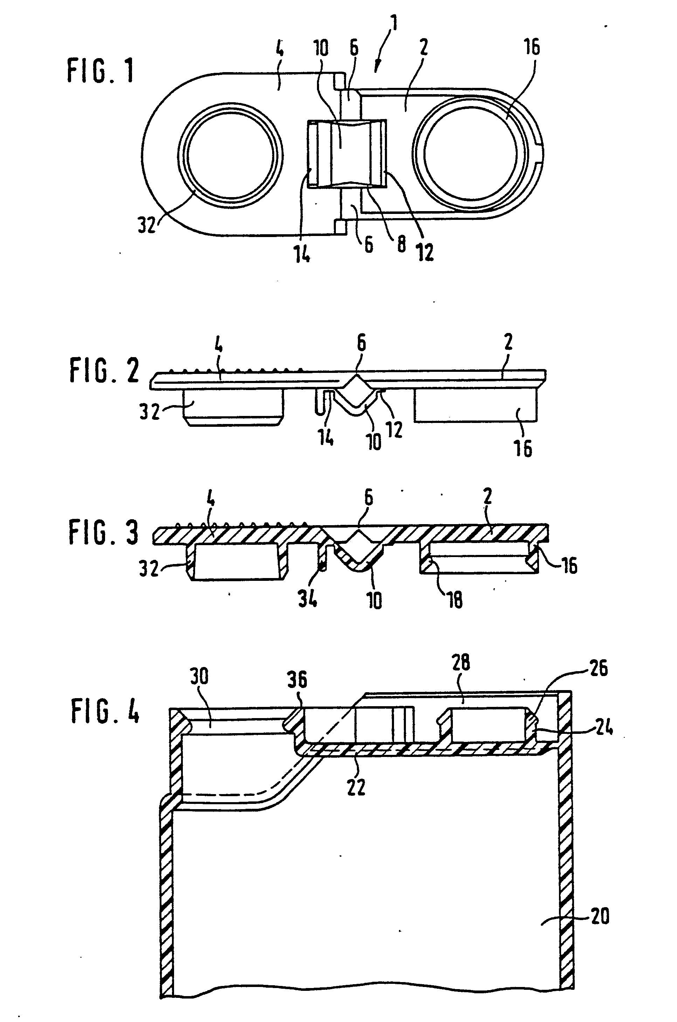

Verschließvorrichtungen dieser Art sind zwar an sich bekannt, u. a. aus dem Dokument FR-A-2 288 683 — L'Oreal. Eine Schwierigkeit bestand aber darin, das Schnappscharnier an einer plattenartig ausgebildeten Verschließvorrichtung zu realisieren. Die Verschließvorrichtung sollte plattenartig sein, damit sie sich in eine Vertiefung in der im Anspruch 1 genannten Wand einschmiegt und nach außen nicht über den Behälter vorsteht. Z. B. steht die Verschließvorrichtung nach Figur 4 von Anderson über die obere Wand des Behälters vor. Dergleichen ist unschön. Durch die Erfindung soll daher erreicht werden, daß die Verschließvorrichtung vollständig innerhalb der Wand verschwindet, so daß ihre obere Fläche in gleicher Ebene liegt wie die obere Fläche der genannten Wand.

Bei einer derart flachen Verschließvorrichtung bestand ein Problem darin, trotzdem die Schnappfunktion zu realisieren, und zwar in nennenswerter Weise. Der Verschlußschenkel sollte nicht aus der Totpunktlage durch eine müde, langweilige Bewegung schließlich auch in Schließstellung gelangen, sondern durch einen echten Schnappvorgang. Beim Vertrieb derartiger Artikel ist es durchaus wesentlich, an den Spieltrieb der Verbraucher zu appellieren. Dann muß ein Schnappvorgang eben ein echter Schnappvorgang sein, sonst erweckt das Produkt nicht die zum Kauf anregende Freude. Es war durchaus nicht zu erwarten, daß es ausreichen würde, Hauptgelenk und Nebengelenke nach Merkmal i von Anspruch 1 an einander gegenüberliegenden Flächen der plattenartigen Scharnierteile anzuordnen. Nach L'Oreal wurden z. B., um die Schnappfunktion sicherzustellen, recht erhebliche Abstände zwischen der Hauptschwenkachse (dort bei 19) und den Nebenachsen (20a und 21) vorgesehen.

Die Verschließvorrichtung nach der Erfindung soll es ermöglichen, auch relativ kompliziert geformte Behälter im Spritzgußverfahren ohne komplizierte Formen herzustellen. Es soll nicht nötig sein, dabei auch auf den Behälter oder seine Überkappe selbst Rücksicht zu nehmen. Da diese getrennt, ebenfalls als Spritzgußteile hergestellt werden können, ist es möglich, sowohl den Behälter wie gegebenenfalls seine Überkappe als auch die Verschließvorrichtung jeweils mit einfachen Formen im Spritzgußverfahren herzustellen. Somit ergibt sich eine kostengünstige Gesamtheit dieser Bestandteile.

Die genannten Bedingungen zugleich werden durch die Erfindung gemäß Anspruch 1 erfüllt.

Es hat sich überraschenderweise herausgestellt, daß eine Anordnung der Achsen nach Merkmal i von Anspruch 1 an gegenüberliegenden Flächen der plattenartigen Teile bereits für die beabsichtigte Schnappfunktion genügte. Auch nach einer Vielzahl von Betätigungen erhält man stets einwandfreie Schnappvorgänge.

Eine plattenartige Verschließvorrichtung nach der Erfindung ermöglicht im Gegensatz zu Anderson eine rationelle Montage mit hoher Geschwindigkeit. Anstelle des zeitraubenden Einschraubens nach Figur 2 und 4 von Anderson genügt ein Aufprellen oder Aufschweißen, wobei zugleich wegen der räumlichen Anordnung für die erforderliche Orientierung und deren Sicherung gesorgt wird.

Erst durch die Gesamtheit dieser Maßnahmen, und die daraus folgenden Produktionsvorteile und Anwendungsvorteile für den Benutzer wird die Verschließvorrichtung nach der Erfindung wirtschaftlich verwertbar.

Es sei noch auf folgende Unterschiede gegenüber der an einer Überkappe sitzenden Verschließvorrichtung von l'Oreal hingewiesen : Dort sind eine Überkappe und ein an dieser angebrachter Verschlußschenkel vorgesehen, sowie das Schnappscharnier. Die zugehörige zu verschließende Entnahmeöffnung ist nun nicht etwa, wie es für eine einwandfreie Funktion sinnvoll wäre, ebenfalls in der Überkappe angeordnet, sondern im Behälter. Die Überkappe hat ein Fenster und einen Ausschnitt, um Zugang zu der Entnahmeöffnung zu verschaffen. Hier fehlt also wiederum die Orientierungssicherung. Je nach Toleranzen der Überkappe und des zugehörigen Aufnahmeabschnitts des Behälters für den Rand der Überkappe dürfte der Verschlußzapfen im allgemeinen neben die Entnahmeöffnung treffen und nur in Sonderfällen ohne Schwierigkeiten eindringen.

Weiterhin ist es bei der Kunstruktion nach l'Oreal nicht möglich, die Verschließvorrichtung in der Außenhaut der Überkappe so verschwinden zu lassen, daß sie nicht als zusätzlicher Bauteil in Erscheinung tritt. Wie die dortige Figur 1 zeigt, entsteht ein recht häßlicher Gegenstand mit einem unorganischen Einschnitt mit Seitenwänden beachtlicher Größe.

Beim Bau der Spritzformen für den Behälter, gegebenenfalls seine Überkappe, und für die Verschließvorrichtung braucht nicht mehr auf die Ausbildung des Scharniers, insbesondere Schnappscharniers, Rücksicht genommen zu werden. Die verschiedenen Bestandteile werden je für sich in speziell dafür hergestellten Formen hergestellt und erst nachträglich zusammengefügt.

Nach Anspruch 2 läßt sich eine besonders günstige Bauform dadurch erzielen, daß das federnde Verbindungsstück des Schnappscharniers in Schließstellung gegen die Wand des Behälters oder der Überkappe gerichtet ist. Es tritt also bei geschlossenem Behälter nach außen nicht störend in Erscheinung. Die Außenfläche des Schnappscharniers bildet in Schließstellung z. B. eine Ebene oder eine dem Behälter oder seiner Überkappe angepaßte gewölbte Fläche, die das Hauptgelenk enthält, so daß sich ein befriedigender ästhetischer Eindruck ergibt. Es stehen keine Scharnierteile nach außen störend vor.

Bei dünneren Platten können nach Anspruch 3 die Nebengelenke an vorspringenden Leisten der Scharnierteile ansetzen.

Der Befestigungsteil der Verschließvorrichtung läßt sich nach Anspruch 4 so ausbilden, daß er durch Kleben oder Schweißen mit der genannten Wand zu verbinden ist.

Nach Anspruch 5 kann der Befestigungsteil mindestens einen Steckteil aufweisen, dessen Kopf sich durch Schweißen mit der Innenseite der Wand verbinden läßt.

Nach Anspruch 6 kann in der Wand eine flache Ausnehmung vorgesehen sein, in der sich eine flache Verschließvorrichtung in ästhetischer Weise unterbringen läßt.

Die Erfindung bezieht sich gemäß einer Weiterbildung nach Anspruch 7 ferner auf einen Behälter für langgestreckte Gegenstände, insbesondere diagnostische Testträger, mit einer Verschließvorrichtung nach einem der vorangehenden Ansprüche.

Bei dieser Weiterbildung der Erfindung werden die Vorteile der erfindungsgemäßen Verschließvorrichtung für einen Behälter eingesetzt, an den im Hinblick auf die zu verpackenden Gegenstände ungewöhnlich hohe Ansprüche gestellt werden.

Die Reaktionsschicht diagnostischer Testträger ist sehr empfindlich gegen Feuchtigkeit. Gleichwohl soll es möglich sein, die Testträger in dem Behälter bis zu mehreren Jahren zu lagern, ohne daß ihre Funktion beeinträchtigt wird. Der Behälter muß also feuchtigkeitsdicht sein und nach der Entnahme von Testträgern wieder feuchtigkeitsdicht zu verschließen sein. Die Handhabung soll einfach sein, da Testträger auch von Nichtmedizinern verwendet werden, z. B. zur Selbstbestimmung des Blutzuckergehaltes. Erwünscht ist (z. B. von Diabetikern) ein Behälter, der z. B. in einer Jackentasche gut mitzuführen ist und eine einfache Entnahme der (meist streifenförmigen) Testträger erlaubt.

Nach Anspruch 7 läßt sich ein Behälter für Testträger schaffen, der diese Anforderungen erfüllt und außerdem kostengünstig herzustellen ist.

Der abgeflachte Querschnitt nach Merkmal d erlaubt ein bequemes Unterbringen des Behälters. Es lassen sich bequem einzelne streifenförmige Testträger dadurch entnehmen, daß man die Packung auf den Kopf stellt. Das abnehmbare Bodenelement nach Merkmal e erlaubt ein schnelles Füllen des Behälters. Nach Merkmal f kann ein Trockenmittel im Bodenelement, statt wie bisher üblich im Verschluß, untergebracht werden. Die Lebensdauer des Trockenmittels wird dadurch verlängert, daß es beim Öffnen des Behälters nicht mehr in vollem Maße der Raumfeuchtigkeit ausgesetzt wird.

Nach Anspruch 8 läßt sich eine hohe mechanische Stabilität und Feuchtigkeitsdichtigkeit des Behälters erzielen.

Nach Anspruch 9 und/oder 10 läßt sich eine wirkungsvolle Dichtung des Verschlusses bei geringen Kosten realisieren.

Ausführungsbeispiele mit weiteren Merkmalen der Erfindung werden im folgenden anhand der Zeichnungen beschrieben.

Figur 1 ist eine Ansicht einer ersten Ausführungsform einer mit einem Schnappscharnier ausgerüsteten Verschließvorrichtung nach der Erfindung, von unten.

Figur 2 ist eine Seitenansicht derselben Verschließvorrichtung.

Figur 3 ist ein mittiger Längsschnitt durch diese Verschließvorrichtung.

Figur 4 ist ein mittiger Längsschnitt durch den oberen Teil eines zugehörigen Behälters.

Figur 5 zeigt die Verschließvorrichtung in Seitenansicht nach Figur 2, jedoch geöffnet.

Figur 6 zeigt eine Verschließvorrichtung nach einer zweiten Ausführungsform der Erfindung in mittigem Längsschnitt.

Figur 7 zeigt die Verschließvorrichtung in Draufsicht von außen (oben).

Figur 8 zeigt in mittigem Längsschnitt eine Überkappe zur Aufnahme einer Verschließvorrichtung nach Figur 6 und 7 und zum Anbringen an einem Behälter.

Figur 9 ist eine Draufsicht auf die Hälfte dieser Überkappe von oben.

Figur 10 zeigt als dritte Ausführungsform perspektivisch einen Behälter zur Aufnahme von Testträgern, ähnlich dem nach Figur 4.

Figur 11 zeigt perspektivisch ein zugehöriges Bodenelement.

Figur 12 ist ein Längsschnitt durch Behälter und Bodenelement nach Figur 10 und 11.

In allen Ausführungsbeispielen beziehen sich Begriffe wie « oben », « unten » und dergleichen immer auf die Lage einer Verschließvorrichtung an einem aufrechtstehenden Behälter, bei dem die Mündung aufwärts gerichtet ist oder an einer auf den Behälter gesteckten Überkappe.

Alle dargestellten Verschließvorrichtungen werden einteilig, z. B. aus Polypropylen gespritzt.

Erste Ausführungsform

Die in Figur 1 bis 3 und 6 dargestellte Verschließvorrichtung 1 hat zwei plattenartige Bestandteile, nämlich einen Befestigungsteil 2 und einen Verschlußschenkel 4, die durch ein als Filmscharnier ausgebildetes Hauptgelenk 6 miteinander verbunden sind. Das Hauptgelenk ist zweiteilig ausgebildet. Zwischen seinen beiden Teilen befindet sich eine Öffnung 8, in der ein federndes Verbindungsstück 10 untergebracht ist. Das Verbindungsstück 10 steht durch zwei Nebengelenke 12 und 14 mit dem Befestigungsteil 2 und dem Verschlußschenkel 4 in Verbindung. Der Befestigungsteil 2 trägt eine Steckhülse 16, die mit einem Innenwulst 18 versehen ist.

Auf der oberen Wand 22 eines Behälters 20 (Figur 4), von dem nur der obere Teil gezeigt ist, befindet sich eine Steckhülse 24 mit einem Außenwulst 26. Der Befestigungsteil 2 läßt sich mit Hilfe seiner Steckhülse 16 auf der Steckhülse 24 des Behälters verankern. Ein die Wand 22 umlaufender Wandteil 28 des Behälters sorgt für eine gleichbleibende Orientierung der Verschließvorrichtung 1.

Der Behälter hat links eine Entnahmeöffnung 30. An dem Verschlußschenkel 4 ist ein dazu passendes Verschließelement 32 in Form eines Rohrstutzens vorgesehen. Am Verschlußschenkel 4 befindet sich ein Vorsprung 34 von der Breite des federnden Verbindungsstücks 10. Der Vorsprung ist so angeordnet, daß er beim Herunterschnappen des Verschlußschenkels 4 einen zugehörigen Anschlag 36, nämlich den Rand der Entnahmeöffnung 30, streifend berührt. Der Verschlußschenkel bleibt auf diese Weise zunächst offen stehen, läßt sich dann aber unter Überwindung des Widerstandes zwischen Vorsprung 34 und Anschlag 36 schließen.

Bei den folgenden Ausführungsformen sind in den Figuren 6ff Bestandteile, die gleiche oder ähnliche Funktion wie die oben beschriebenen haben mit den oben verwendeten Bezugszeichen versehen, denen jedoch eine 100er- oder 500er-Stelle vorangesetzt wurde.

Zweite Ausführungsform

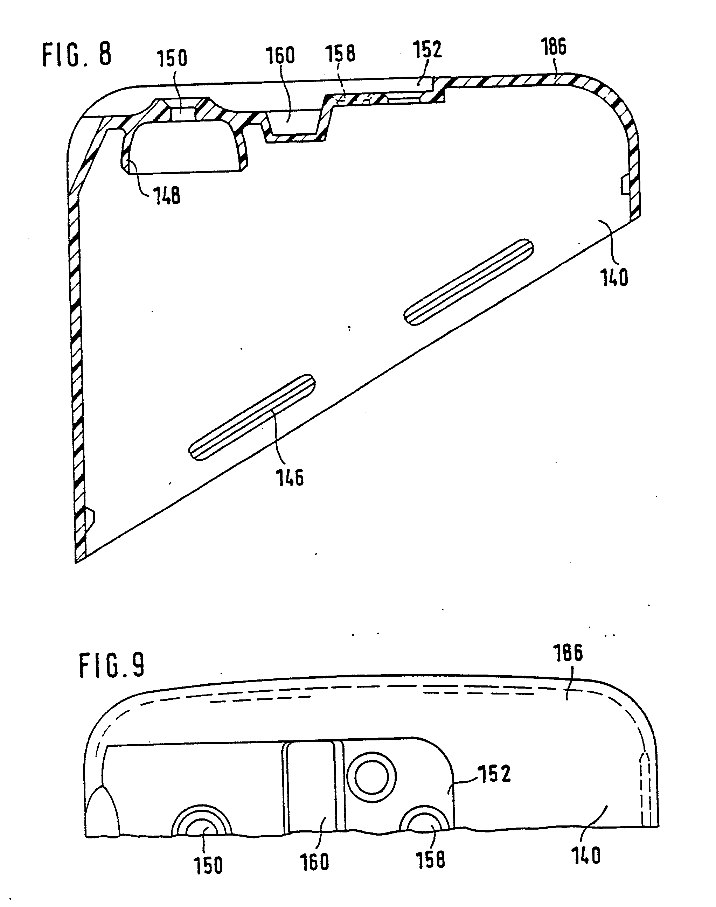

Die Figuren 6 und 7 zeigen ein Schnappscharnier 101, das nicht unmittelbar an einem Behälter sondern an einer Überkappe 140 nach Figur 8 und 9 anzubringen ist, die ihrerseits auf einem Behälter zu befestigen ist. Während das federnde Verbindungsstück 10 bei der ersten Ausführungsform gewinkelt ist, ist es hier (110) bogenförmig ausgebildet. Es hat aber die gleiche Funktion. Die beiden Nebengelenke 112 und 114 sitzen nach Figur 6 nicht unmittelbar am Befestigungsteil 102 und Verschlußschenkel 104 sondern an Leisten 142 und 144, die nach unten vorspringen. Der Verschlußschenkel 104 ist durch seitliche Wandstreifen 133 versteift.

Die in Figur 8 und 9 dargestellte Überkappe 140 hat eine schräge untere Berandung. Die Überkappe hat nahe ihrem unteren Rand Schnappwülste 146, die in entsprechende Vertiefungen eines nicht dargestellten Behälters nach dem Aufstecken einrasten. Die Überkappe hat oben links innen einen Napf 148, der die Behältermündung umgeben soll, und zentrisch dazu eine Austrittsöffnung 150. An der Verschließvorrichtung 101 ist ein dazu passendes Verschließelement 132 in Form eines hohlen Zapfens vorgesehen.

Die Überkappe 140 hat oben eine flache Ausnehmung 152 zur Aufnahme der Verschließvorrichtung 101. An dem Befestigungsteil 102 sind drei Steckzapfen 154 vorgesehen, die unten je einen äußeren Ringwulst 156 haben. Die Steckzapfen lassen sich in durchgehende Löcher 158 der Überkappe 140 eindrücken, sind durch ihre Ringwülste 156 gegen Herausrutschen und Herausziehen gesichert und halten den Befestigungsteil 102 in der flachen Ausnehmung 152 der Überkappe fest.

Zur Unterbringung des federnden Verbindungsstückes 110 ist in der oberen Wand 186 der Überkappe eine Vertiefung in Form einer quer verlaufenden Rinne 160 vorgesehen.

Die Offenstellung der Verschließvorrichtung ähnelt derjenigen nach Figur 5.

Die Steckzapfen 154 können nach dem Eindrücken in die Löcher 158 zur größeren Sicherheit auch mit der Unterseite der von den Löchern durchbrochenen oberen Wand 186 verschweißt werden. Stattdessen kann der Befestigungsteil 2, 102 ohne Steckzapfen mit der darunterliegenden Wand flächig oder punktweise verschweißt oder verklebt werden.

Dritte Ausführungsform

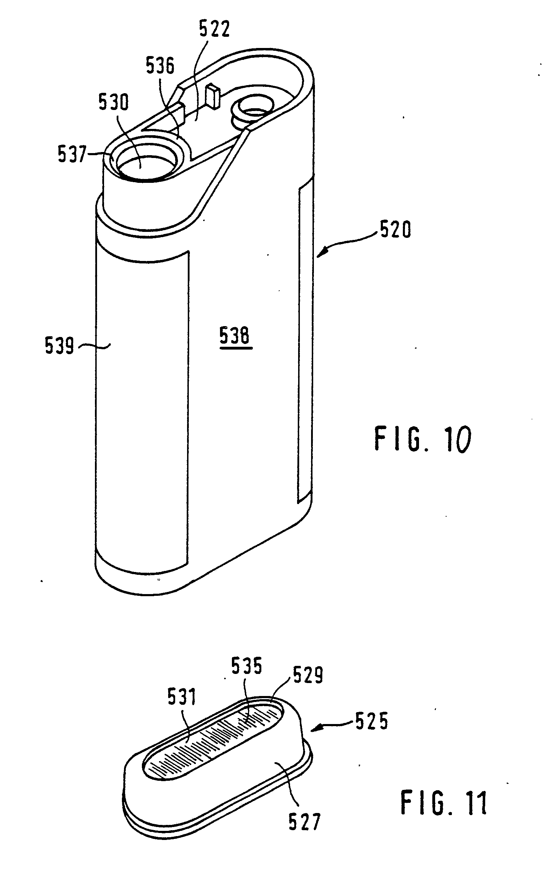

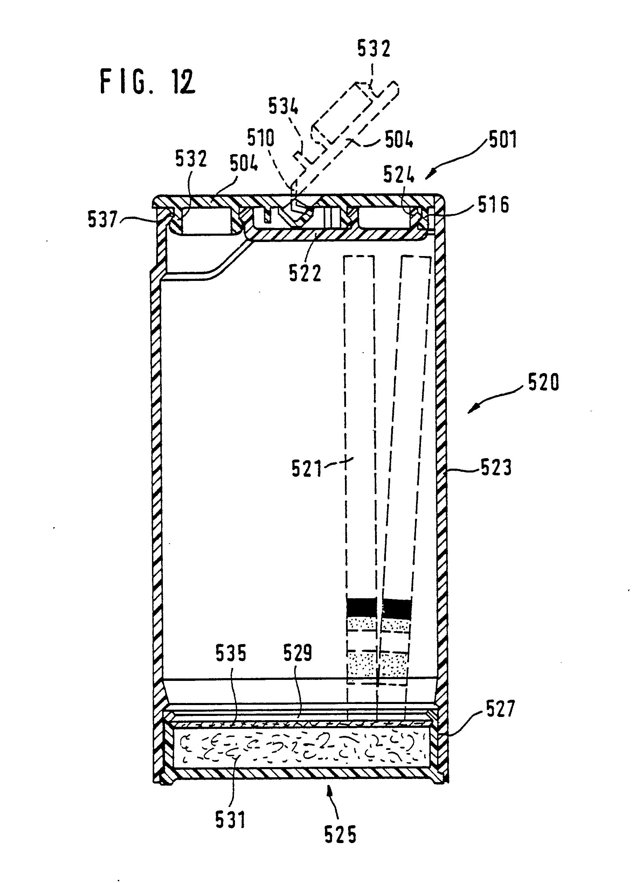

Figur 10 bis 12 zeigen eine Variante der ersten Ausführungsform, nämlich einen Behälter 520 für eine Vielzahl von Testträgern 521, von denen nur zwei dargestellt sind. Der Behälter hat einen Behälterkörper 523 und eine Verschließvorrichtung 501, wie sie im Prinzip in den Figuren 1 bis 3 dargestellt ist.

In das untere, offene Ende des Behälterskörpers 523 ist ein Bodenelement 525 einsteckbar. Die Innenwandung des unteren Endes des Behälterkörpers und die Außenwandung 527 des Bodenelements sind so einander angepaßt, daß das Bodenelement feuchtigkeitsdicht in den Behälterkörper einsteckbar ist. Die Außenwandung des Bodenelements kann mit der Innenwandung des unteren Endes des Behälterkörpers durch Ultraschallschweißung luftdicht und damit auch feuchtigkeitsdicht verbunden werden.

Das Bodenelement umschließt bis auf eine Einfüllöffnung 529 eine Kammer 531, die zur Aufnahme eines Trockenmittels dient. Die Kammer ist gegen den Innenraum des Behälters 520 mit einem die Feuchtigkeitsaufnahme regulierenden Element, beispielsweise einer Kartonscheibe 535 verschlossen.

Wie Figur 10 zeigt, ist der Behälter 520 flach. Er hat parallele Seitenwände 538 und abgerundete Endseiten 539. Er könnte auch einen elliptischen Querschnitt haben. Die Entnahmeöffnung 530 ist etwa konzentrisch zu der Rundung der einen Endseite angeordnet. Hierdurch wird die Entnahme von Testträgern vereinfacht. Die Packung braucht hierzu nur in Richtung der Entnahmeöffnung gekippt zu werden.

Ein zu diesem Behälter passender Verschluss ist in den Figuren 1 bis 3 dargestellt und wurde oben im ersten Ausführungsbeispiel beschrieben. Die Deckwand 522, die die obere Endfläche des Behälterkörpers darstellt, ist ein integraler Bestandteil des Behälterkörpers. Die einzige Öffnung des Behälterkörpers nach oben ist die kreisrunde Entnahmeöffnung 530. Diese läßt sich durch das Verschließelement 532, das am Verschlußschenkel 504 sitzt, verschließen. Das Verschließelement 532 hat hier (im Gegensatz zu dem nach Figur 2 und 3) die Form eines Ringes, dessen Außenwandung im Querschnitt kreisbogenförmig ist. Diese Außenwandung bildet die Dichtfläche, die im Schließzustand dichtend an einem Innenwulst 537 der Entnahmeöffnung 530 anliegt. Die obere Wandung dieses Innenwulstes verläuft konisch. Sie verjüngt sich gegen das Behälterinnere. Der Innenwulst und die ihn umgebenden Wandteile sind gemeinsam relativ dick und geben beim Verschließen nur wenig nach. Dagegen ist das Verschließelement 532 verhältnismäßig dünnwandig und wird beim Schließen geringfügig gegen das Zentrum der Öffnung zusammengedrückt. Die kreisrunde Form der Entnahmeöffnung 530 und des Verschließelements 532 führen hier zu einem günstigen Ringspannverhalten und damit zu einer guten Abdichtung.

Die Funktion des Vorsprunges 534 des Verschlußschenkels 504 wurde schon im ersten Ausführungsbeispiel beschrieben. Es ist wichtig, daß ein Behälter mit feuchtigkeitsempfindlichem Inhalt stets vollständig geschlossen wird. Durch den Anschlag des Vorsprunges 534 an der Berandung der Entnahmeöffnung (Anschlag 536) wird dem Benutzer kenntlich gemacht, daß der Behälter nicht geschlossen ist, was weniger deutlich zu erkennen wäre, wenn der Verschlußschenkel 504 weiter zuklappen würde. Der Benutzer wird also genötigt, den Verschlußschenkel vollständig niederzudrücken.

## Patentansprüche

1. Verschließvorrichtung (1 usw.) in Form eines gesonderten Kunststoff-Spritzteiles zum Verschließen und Öffnen der Entnahmeöffnung eines Behälters oder einer Überkappe des Behälters, wobei

a) die Verschließvorrichtung zur direkten Befestigung an der die Entnahmeöffnung enthaltenden Wand (22, 186, 522) des Behälters oder der Überkappe eingerichtet ist,

b) die Verschließvorrichtung (1 usw.) zwei durch ein Scharnier miteinander verbundene plattenartige Scharnierteile hat, nämlich:

b1) einen Befestigungsteil (2 usw.) zum dauerhaften Befestigen an der Wand,

b2) einen Verschlußschenkel (4, usw.) mit einem zur Entnahmeöffnung passenden Verschließelement (32 usw.),

gekennzeichnet durch folgende Merkmale:

c) der Befestigungsteil und der zugehörige Abschnitt der Wand sind für eine orientierungssichere Befestigung eingerichtet,

d) das Scharnier ist ein Schnappscharnier, bei dem die beiden Scharnierteile zunächst durch ein Hauptgelenk (6 usw.) miteinander verbunden sind,

e) mindestens ein bogen- oder winkelförmiges, federndes Verbindungsstück (10 usw.) ist an seinen beiden Enden durch Nebengelenke (12, 14 usw.) mit beiden Scharnierteilen verbunden,

f) die Nebengelenke haben quer zu den Gelenkachsen je einen Abstand vom Hauptgelenk,

g) Hauptgelenk und Nebengelenke sind als Filmscharniere ausgebildet,

h) derart, daß die Scharnierteile die Tendenz haben, aus einer Stellung labilen Gleichgewichts, die sich innerhalb ihres Schwenkbereichs befindet, in die eine oder andere von zwei Endstellungen zu schnappen,

i) das Hauptgelenk (6 usw.) einerseits und die Nebengelenke (12, 14 usw.) andererseits befinden sich an einander gegenüberliegenden Flächen der beiden plattenartigen Scharnierteile. (Figuren 2, 3, 6, 7)

2. Verschließvorrichtung nach Anspruch 1, gekennzeichnet durch folgende Merkmale:

a) das federnde Verbindungsstück (10 usw.) weist in Schließstellung der an der Wand angebrachten Verschließvorrichtung gegen die Wand,

b) das Hauptgelenk (6 usw.) ist an der von der Wand abgekehrten Seite des Schnappscharniers angeordnet. (Figur 1 bis 7)

3. Verschließvorrichtung nach Anspruch 1 oder 2, dadurch gekennzeichnet, daß die Nebengelenke (12, 14 usw.) an Leisten (142, 144) sitzen, die von den Scharnierteilen gegen den Behälter vorspringen. (Figur 6)

4. Verschließvorrichtung nach Anspruch 1, dadurch gekennzeichnet, daß der Befestigungsteil (2 usw.) derart ausgebildet ist, daß er mit der Wand durch Kleben oder Schweißen zu verbinden ist.

5. Verschließvorrichtung nach Anspruch 1, dadurch gekennzeichnet, daß der Befestigungsteil zur Verbindung mit der Wand mindestens einen Steckteil (Steckzapfen 154) aufweist, dessen Kopf so ausgebildet ist, daß er sich durch Schweißen mit der Innenseite der Wand verbinden läßt.

6. Verschließvorrichtung nach einem der vorangehenden Ansprüche, dadurch gekennzeichnet, daß die Wand (186) eine flache Ausnehmung (152) zur Aufnahme der Verschließvorrichtung hat.

7. Behälter für langgestreckte Gegenstände, insbesondere diagnostische Testträger, mit einer Verschließvorrichtung nach einem der vorangehenden Ansprüche, wobei

a) ein länglicher Behälterkörper ein Entnahmeende hat, an dem sich die Entnahmeöffnung (530) befindet und einen Boden an seinem entgegengesetzten Ende,

b) der Verschlußschenkel (504) ein Verschließelement (532) mit einer einem Dichtrand der Entnahmeöffnung angepaßten Dichtfläche hat,

c) die Querschnittsfläche der Entnahmeöffnung (530) erheblich kleiner ist als die Querschnittsfläche des Behälterkörpers, gekennzeichnet durch folgende Merkmale :

d) der Behälterkörper hat in einer Ebene quer zu seiner Längsrichtung einen abgeflachten Querschnitt,

e) in den Behälterkörper ist ein Bodenelement (525) einsetzbar, das mit dem Behälterkörper feuchtigkeitsdicht zu verbinden ist,

f) das Bodenelement hat eine Kammer für ein Trockenmittel,

g) die Kammer ist gegen das Innere des Behälters (520) durch ein die Flüssigkeitszufuhr regulierendes Element verschlossen.

8. Behälter nach Anspruch 7, dadurch gekennzeichnet, daß Behälterkörper und Bodenelement eine Wandstärke von mindestens etwa 1 mm haben und aus Polyethylen oder Polypropylen bestehen.

9. Behälter nach Anspruch 7, gekennzeichnet durch folgende Merkmale :

a) das Verschließelement (532) ist ein ringförmiger Vorsprung, der vom Verschlußschenkel senkrecht vorsteht,

b) der Vorsprung ist gegen sein Zentrum elastisch komprimierbar,

c) die Dichtfläche hat einen kreisbogenförmigen Querschnitt.

10. Behälter nach Anspruch 7 oder 8, dadurch gekennzeichnet, daß das Bodenelement (525) mit der zugehörigen Öffnung des Behälterkörpers durch Ultraschallschweißung luft- und feuchtigkeitsdicht verbunden ist.

**Claims**

1. Closure device (1 etc.) in the form of a separate injection moulded plastic component for closing and opening the removal opening of a container or of an overcap of the container, in which

a) the closure device is adapted for direct connection to the wall (22, 186, 522) including the removal opening of the container or of the overcap,

b) the closure device (1 etc.) has two plate-like hinge members connected together by means of a hinge, namely :

b1) a fastening member (2 etc.) for permanent connection to the wall,

b2) a closure limb (4 etc.) with a closure element (32 etc.) fitting the removal opening, characterised by the following features :

c) the fastening member and the associated section of the wall are adapted to be connected in the correct orientation,

d) the hinge is a snap hinge in which the two hinge members are firstly connected together by a main joint (6 etc.),

e) at least one arcuate or angular, resilient connecting member (10 etc.) is connected at its two ends to both hinge members by subsidiary joints (12, 14 etc.),

f) the subsidiary joints are each spaced from the main joint transversely to the joint axes,

g) the main joint and the subsidiary joints are constructed as integral hinges,

h) in such a manner that the hinge members have the tendency to snap out of a position of unstable equilibrium, which is situated within their pivotal range, into one or other of two final positions, i) the main joint (6 etc.) on the one hand and the subsidiary joints (12, 14 etc.) on the other hand are situated on opposing surfaces of the two plate-like hinge members. (Figures 2, 3, 6, 7)

2. Closure device as claimed in claim 1, characterised by the following features :

a) the resilient connecting member (10 etc.) is directed towards the wall in the closed position of the closure device mounted on the wall,

b) the main joint (6 etc.) is arranged on the side of the snap hinge directed away from the wall. (Figures 1 to 7)

3. Closure device as claimed in claim 1 or 2, characterised in that the subsidiary joints (12, 14 etc.) are on bars (142, 144) which project from the hinge members towards the container. (Figure 6)

4. Closure device as claimed in claim 1, characterised in that the securing member (2 etc.) is so constructed that it may be connected to the wall by adhesive or by welding.

5. Closure device as claimed in claim 1, characterised in that the securing member for connecting to the wall has at least one plug member (plug pin 154) whose head is so constructed that it may be connected to the inner side of the wall by welding.

6. Closure device as claimed in one of the preceding claims, characterised in that the wall (186) has a flat recess (152) for receiving the closure device.

7. Container for elongate articles, particularly diagnostic test carriers, with a closure device as claimed in one of the preceding claims, in which

a) an elongate container body has a removal end in which the removal opening (530) is situated and a bottom at its opposite end,

b) the closure limb (504) has a closure element (532) with a sealing surface matched to a sealing edge of the removal opening,

c) the cross-sectional area of the removal opening (530) is considerably smaller than the cross-sectional area of the container body, characterised by the following features. :

d) the container body has a flattened cross-section in a plane transverse to its longitudinal direction,

e) a bottom element (525), which is to be connected in a moisture-tight manner to the container body, may be inserted into the container body,

f) the bottom element has a chamber for a drying agent,

g) the chamber is sealed from the interior of the container (520) by an element regulating the supply of liquid.

8. Container as claimed in claim 7, characterised in that the container body and bottom element have a wall thickness of at least about 1 mm and comprise polyethylene or polypropylene.

9. Container as claimed in claim 7, characterised by the following features :

a) the closure element (532) is an annular projection which projects perpendicularly from the closure limb,

b) the projection is elastically compressible towards its centre,

c) the sealing surface has a circularly arcuate cross-section.

10. Container as claimed in claim 7 or 8, characterised in that the bottom element (525) is connected in a moisture and air-tight manner to the associated opening in a container body by ultrasonic welding.


**Revendications**

1. Dispositif de fermeture (1 etc.) sous la forme d'une pièce séparée en matière plastique moulée par injection pour fermer et ouvrir l'ouverture de déchargement d'un récipient ou d'un capuchon du récipient, dans lequel :

a) le dispositif de fermeture est agencé pour être fixé directement sur la paroi (22, 186, 522) du récipient ou du capuchon qui comprend l'ouverture de déchargement,

b) le dispositif de fermeture (1 etc.) comporte deux parties articulées en forme de plaques reliées entre elles par une charnière, à savoir :

b1) une partie de fixation (2 etc.) pour la fixation permanente sur la paroi,

b2) une aile de fermeture (4 etc.) avec un élément de fermeture (32 etc.) adapté à l'ouverture de déchargement,
caractérisé par les caractéristiques suivantes :

c) la partie de fixation et la section correspondante de la paroi sont agencées pour une fixation assurant leur orientation,

d) la charnière est une charnière à déclic dans laquelle les deux parties articulées sont tout d'abord reliées entre elles par une articulation principale (6 etc.),

e) au moins une pièce de liaison élastique (10 etc.) en forme d'arc ou d'angle est reliée à ses deux extrémités par des articulations secondaires (12, 14 etc.) aux deux parties articulées,

f) les articulations secondaires sont chacune à distance de l'articulation principale perpendiculairement à l'axe d'articulation,

g) l'articulation principale et les articulations secondaires sont réalisées sous la forme de charnières-films,

h) de telle façon que les parties articulées aient tendance à sauter dans l'une ou l'autre de deux positions finales à partir d'une position d'équilibre instable qui se trouve à l'intérieur de leur zone de pivotement,

i) l'articulation principale (6 etc.), d'une part, et les articulations secondaires (12, 14, etc.), d'autre part, se trouvent sur des surfaces en face l'une de l'autre des deux parties articulées en forme de plaque. (Figures 2, 3, 6, 7).

2. Dispositif de fermeture selon la revendication 1, caractérisé par les caractéristiques suivantes :

a) la pièce de liaison élastique (10 etc.) est dirigée vers la paroi dans la position de fermeture du dispositif de fermeture fixé à la paroi.

b) l'articulation principale (6 etc.) est disposée sur le côté de la charnière à déclic qui est opposé à la paroi. (Figures 1 à 7).

3. Dispositif de fermeture selon la revendication 1 ou 2, caractérisé par le fait que les articulations secondaires (12, 14, etc.) sont fixées sur des nervures (142, 144) qui font saillie vers le récipient à partir des parties articulées (Figure 6).

4. Dispositif de fermeture selon la revendication 1, caractérisé par le fait que la partie de fixation (2 etc.) est conformée de telle façon qu'elle puisse être reliée à la paroi par collage ou soudage.

5. Dispositif de fermeture selon la revendication 1, caractérisé par le fait que la partie de fixation, pour sa liaison avec la paroi, comporte au moins une partie à enfoncer (téton à enfoncement 154) dont la tête est conformée de telle façon qu'elle puisse être reliée par soudage au côté intérieur de la paroi.

6. Dispositif de fermeture selon la revendication 1, caractérisé par le fait que la paroi (186) comporte un évidement plat (152) pour recevoir le dispositif de fermeture.

7. Récipient pour des objets allongés, en particulier des supports de réactif à usage diagnostique, avec un dispositif de fermeture selon l'une des revendications précédentes, dans lequel

a) un corps de récipient allongé comporte une extrémité de déversement sur laquelle se trouve l'ouverture de déchargement (530), et un fond à son extrémité opposée,

b) l'aile de fermeture (504) comporte un élément de fermeture (532) comprenant une surface d'étanchéité ajustée à un bord d'étanchéité de l'ouverture de déchargement,

c) la surface de la section transversale de l'ouverture de déchargement (530) est nettement plus petite que la surface de la section transversale du corps du récipient,
caractérisé par les caractéristiques suivantes :

d) le corps du récipient comporte une section transversale aplatie dans un plan perpendiculaire à sa direction longitudinale,

e) dans le corps du récipient, on introduit un élément de fond (525) qui peut être relié au corps du récipient de manière étanche à l'humidité,

f) l'élément de fond comporte une chambre pour un déshydrateur,

g) la chambre est fermée par rapport à l'intérieur du récipient (520) par un élément régulateur de l'amenée d'humidité.

8. Récipient selon la revendication 7, caractérisé par le fait que le corps du récipient et l'élément de fond ont une épaisseur de paroi d'au moins 1 mm environ et sont constitués de polyéthylène ou de polypropylène.

9. Récipient selon la revendication 7, caractérisé par les caractéristiques suivantes :

a) l'élément de fermeture (532) est une saillie annulaire qui fait saillie perpendiculairement depuis l'aile de fermeture,

b) la saillie peut être comprimée élastiquement vers son centre,

c) la surface d'étanchéité présente une section transversale en arc de cercle.

10. Récipient selon la revendication 7 ou 8, caractérisé par le fait que l'élément de fond (525) est relié à l'ouverture correspondante du corps du récipient de manière étanche à l'air et à l'humidité par soudage par ultrasons.

FIG. 1

FIG. 2

FIG. 3

FIG. 4

FIG. 5

FIG. 6

FIG. 7

FIG. 8

150    160    158    152    186

148

140

146

FIG.9

186

152

150    160    158    140

522

536

530

537

520

539

538

FIG. 10

535 529

531

525

527

FIG. 11

FIG. 12